# EUROPÄISCHE PATENTSCHRIFT

(11) **EP 3 154 373 B1**
(45) Veröffentlichungstag und Bekanntmachung des Hinweises auf die Patenterteilung: **10.10.2018**
(21) Anmeldenummer: 15734055.5
(22) Anmeldetag: 03.06.2015
(51) Int. Cl.: A23L 5/20, G01N 33/02

(54) **VERFAHREN ZUR EXTRAKTION VON KONTAMINANTEN AUS AGRARISCHEN PRODUKTEN SOWIE VORRICHTUNG HIERFÜR**
METHOD FOR EXTRACTING CONTAMINANTS FROM AGRARIAN PRODUCTS AND APPARATUS THEREFOR
PROCÉDÉ POUR EXTRAIRE DES CONTAMINANTS CONTENUS DANS DES PRODUITS AGRICOLES ET DISPOSITIF POUR METTRE LEDIT PROCÉDÉ EN OEUVRE

(30) Priorität: 12.06.2014 AT 2512014 U
(43) Veröffentlichungstag der Anmeldung: 19.04.2017
(73) Patentinhaber: Erber Aktiengesellschaft, 3131 Getzersdorf bei Traismauer (AT)
(72) Erfinder: BINDER, Eva-Maria, A-3430 Tulln (AT); CVAK, Barbara, A-1140 Wien (AT); SCHIESSL, Alois, A-1160 Wien (AT); HÄUBL, Georg, A-1050 Wien (AT)
(74) Vertreter: Cunow, Gerda
(86) Internationale Anmeldenummer: PCT/AT2015/000083
(87) Internationale Veröffentlichungsnummer: WO 2015/188205

(56) Entgegenhaltungen:
- US-A1- 2005 142 254
- US-A1- 2005 287 682
- RUDOLF KRSKA ET AL: "Rapid test strips for analysis of mycotoxins in food and feed", ANALYTICAL AND BIOANALYTICAL CHEMISTRY, SPRINGER, BERLIN, DE, Bd. 393, Nr. 1, 21. Oktober 2008 (2008-10-21), Seiten 67-71, XP019652963, ISSN: 1618-2650

## Beschreibung

Die vorliegende Erfindung bezieht sich auf ein Verfahren zur Extraktion von Kontaminanten aus agrarischen Produkten oder agrarische Produkte enthaltenden Lebens- oder Futtermitteln, umfassend ein in Kontakt bringen eines Lösungsmittels für die Kontaminanten mit den die Kontaminanten angelagert aufweisenden bzw. enthaltenden agrarischen Produkten oder den Lebens- oder Futtermitteln, sowie wenigstens einen Extraktionspuffer in einem gegebenenfalls verschließbaren Mischbehälter, sowie eine Vorrichtung zum Extrahieren von Kontaminanten aus agrarischen bzw. von der Oberfläche agrarischer Produkte(n) oder agrarische Produkte enthaltenden Leberis- oder Futtermitteln, umfassend einen gegebenenfalls verschließbaren Mischbehälter.

Agrarische Produkte oder agrarische Produkte enthaltende Lebens- oder Futtermittel sind häufig mit Kontaminanten, wie Mykotoxinen, Allergenen oder GVO (genetisch veränderten Organismen) verunreinigt oder beladen, welche Verunreinigungen bzw. Beladungen mit Mykotoxinen, Allergenen oder GVO nicht nur zu bedeutenden wirtschaftlichen Einbußen führen, sondern vor allem auch nicht unbeträchtliche gesundheitliche Beeinträchtigungen bei Letztverbrauchern verursachen können. Besonders gefährlich sind die Mykotoxine insbesondere deshalb, da sie eine überaus hohe Stabilität gegenüber chemischen und physikalischen Einflüssen, wie z.B. Temperatur, aufweisen, so dass sie auch nach der Verarbeitung der agrarischen Produkte zu Lebensmitteln oder Futtermitteln häufig noch im Wesentlichen in unveränderten Konzentrationen nachgewiesen werden können und ihre schädlichen Einflüsse auf die Letztverbraucher ausüben können. Allergene Kontaminanten können bei Personen, die unter entsprechenden Allergien leiden, allergischer Reaktionen bis hin zum anaphylikatischen Schock auslösen. Die Beimengung von GVO agrarischen Produkten in Nahrungsmittel ist in vielen Ländern gesetzlich verboten und es muss vom Nahrungsmittelhersteller sichergestellt sein, dass es keine Kontaminationen davon im Lebensmittel gibt.

Betreffend die schädlichen Wirkungen von Mykotoxinen wurde bei einigen von ihnen eine karzinogene Wirkung festgestellt, andere führen zu Entwicklungsstörungen von Individuen, welchen mit Mykotoxinen verunreinigte Lebens- oder Futtermittel verabreicht wurden oder sie zeigen beispielsweise eine starke östrogene Wirkung, weshalb sie hormonelle Störungen verursachen können oder schließlich wurde festgestellt, dass sie bei Tieren auch zu einer Erhöhung der Fehlgeburtenrate beitragen können.

Auch weitere Kontaminanten, wie genetisch veränderte Organismen (GVO) können nachteilige Wirkungen haben, wobei diese häufig noch nicht vollständig bekannt sind, da Langzeitstudien oft noch existieren bzw. noch nicht abschlossen sind.

So stellt es ein weiteres Problem, beispielsweise bei Anlieferung von agrarischen Produkten dar, zu untersuchen, ob eine gelieferte Charge frei von unerwünschten Kontaminanten, wie Mykotoxinen oder GVO ist oder ob und gegebenenfalls welche Menge und Arten von Kontaminanten, wie Mykotoxine(n) oder GVO darin enthalten sind. Unerwünschte Kontaminanten werden bei herkömmlichen Verfahren von ihrer Analytik aus den agrarischen Produkten mit organischen Lösungsmitteln herausgelöst, wie beispielsweise Ethanol, Methanol, Ethylacetat, Chloroform, Acetonitril oder deren wässrige Lösungen. So werden derzeit vor allem in den USA und auch Europa bei Getreidesilos üblicherweise Proben der angelieferten Getreide gezogen, wofür das Getreide gemahlen wird und anschließend auf die unterschiedlichsten Parameter getestet wird. Problematisch sind derartige Voruntersuchungen nicht nur dahingehend, dass bei Lagerstätten für agrarische Produkte üblicherweise keine bzw. nur sehr eingeschränkte Laboratorien und Ausrüstungen für den Nachweis von schädlichen Substanzen vorhanden sind, sei es aus Kostengründen, sei es aufgrund der Tatsache, dass derartige Silos üblicherweise keine Großbetriebe darstellen, in welchen Fachpersonal und Einrichtungen für den Nachweis von schädlichen Substanzen durchgehend anwesend sind bzw. vorrätig gehalten werden können, sondern vor allem dahingehend, dass das Getreide von den angelieferten LKW's erst dann abgeladen werden darf, wenn alle erforderlichen Tests durchgeführt wurden und die gesetzlich vorgeschriebenen Grenzwerte nicht überschritten werden.

Die Untersuchungen werden noch dadurch erschwert, dass es eine Vielzahl von verschiedensten Mykotoxinen gibt, welche nicht alle mit demselben (denselben) Lösungsmittel(n) bzw. Lösungsmittelgemisch(en) bzw. Extraktionsmittelgemisch(en) von den agrarischen Produkten extrahiert werden können, so dass alleine für einen Test, beispielsweise bei einem Getreidesilo, eine Vielzahl von verschiedensten Lösungsmitteln, Nachweisstreifen und dgl. vorrätig gehalten werden muss, um Vorabtests in Bezug auf eine mögliche bzw. in Bezug auf eine konkrete Verunreinigung mit Mykotoxinen durchführen zu können. Als Folge davon müssen LKW's die Getreidechargen führen, vor ihrem Abladen verschiedenen Tests unterzogen werden, damit die gegebenenfalls mit Mykotoxinen beladenen agrarischen Produkte nicht in die Nahrungs- und Futtermittelindustrie gelangen, wodurch die Dekontamination insgesamt teurer wird. Aus den oben genannten Gründen ist es somit besonders wichtig, dass die gesamte Prozedur der Tests möglichst einfach und schnell sowie an möglichst kleinen Chargen durchgeführt werden kann, da, erst wenn alle Tests durchgeführt sind und die gesetzlichen Grenzwerte nicht überschritten sind, das Getreide von einem LKW abgeladen werden darf.

US 2005/0142254 offenbart ein Verfahren zur Extraktion von Kontaminanten aus agrarischen Produkten, die Methode umfassend ein in Kontakt bringen eines Lösungsmittel für die Kontaminanten mit den agrarischen Produkt, sowie einen Extraktionspuffer mit einem Hämin und/oder Häm-haltige Substanz.

Der US 2005/0287682 A1 ist ein Verfahren zu entnehmen, in welchem ein Behälter, wie eine Petri-Schale, eine Mikrowell oder ein Röhrchen mit einer ersten Substanz befüllt wird und in der Folge über dieser ersten Substanz ein leicht auflösbarer Film im Inneren des Behälters angeordnet wird, welcher Film die erste Substanz gegenüber der Umwelt oder gegebenenfalls weiteren Substanzen im Inneren des Behälters abschließt.
Die vorliegende Erfindung zielt nun darauf ab, ein Verfahren zur Verfügung zu stellen, mit welchem es gelingt, Mykotoxine aus agrarischen Produkten bzw. agrarische Produkte enthaltenen Lebens- bzw. Futtermitteln zuverlässig quantitativ zu extrahieren, ohne dass gleichzeitig für Letztverbraucher gefährliche bzw. Schädliche Substanzen, wie organische Lösungsmittel oder andere gefährliche Reaktionsmittel in die Proben eingebracht werden. Zur Lösung dieser Aufgabe wird das erfindungsgemäße Verfahren im Wesentlichen so geführt, dass der in einer in dem Lösungsmittel Iöslichen Folie enthaltene, mit wenigstens einem insbesondere nicht ionischen Tensid versetzte Extraktionspuffer sowie das kontaminierte agrarische Produkt oder Lebens- oder Futtermittel mit kaltem Wasser als Lösungsmittel in dem Mischbehälter in Kontakt gebracht werden, und der Extraktionspuffer sowie die Kontaminanten durch Schütteln oder Rühren in dem Mischbehälter in Lösung gebracht werden. Indem der in einem Lösungsmittel löslichen Folie enthaltene mit wenigstens einem Tensid versetzte Extraktionspuffer durch Schütteln oder Rühren mit Wasser in einem Mischbehälter in Lösung gebracht wird, gelingt es, die Zahl der vorrätig zu haltenden Reagenzien so weit als möglich herabzusetzen und insbesondere kann mit einer derartigen Verfahrensführung sichergestellt werden, dass ein fachlich geschultes Personal, insbesondere in kleinen Betrieben, nicht bei derartigen Nachweisen zugegen sein muss, da das Auflösen eines Beutels, in welchem sämtliche für ein Extrahieren von Kontaminanten geeignete Lösungsmittel enthalten sind, auch einem Kind bzw. Anfänger des Segelsports ohne weiters zuzutrauen ist.
Indem welterhin das Verfahren so geführt wird, dass auch die Nahrungs- bzw. Futtermittel mit dem kalten Wasser als Lösungsmittel in dem Mischbehälter in Kontakt gebracht werden, gelingt es in ein und demselben Reaktionsgefäß, insbesondere einem Becher, sämtliche Bestandteile zu vermischen, worauf die an den agrarischen Produkten angelagerten Mykotoxine quantitativ aufgelöst bzw. heruntergelöst werden und in der Folge unmittelbar, beispielsweise durch Anwendung eines Schnelltests die Frage geklärt werden kann, ob und welche Mengen an Mykotoxin an der Oberfläche der agrarischen Produkte enthalten sind.

indem, wie dies einer Weiterbildung des erfindungsgemäßen Verfahrens entspricht, das Verfahren so geführt wird, dass Wasser mit einer Temperatur zwischen 8 °C und 25 °C, insbesondere destilliertes Wasser eingesetzt wird, wird überdies vermieden, Heizeinrichtungen bzw. Vorrichtungen, mit welchen Wasser erwärmt werden kann, einzusetzen, so dass auch der Energieaufwand ebenso wie der Zeitaufwand, welcher für die Durchführung des Verfahrens erforderlich ist, extrem niedrig gehalten werden können. Auch wenn das Verfahren mit Leitungswasser voll funktionsfähig ist, ist insbesondere, wenn sichergestellt werden soll, dass nicht gegebenenfalls mit den Lösungsmitteln Schad- oder Störstoffe, wie z.B. Nitrate eingebracht werden, es bevorzugt, destilliertes Wasser einzusetzen.

Wenn, wie dies einer Welterbildung der Erfindung entspricht, als Extraktionspuffer wenigstens ein Puffer gewählt aus der Gruppe der Borat-Puffer mit einem pH von 8,5, Carbonat-Puffer mit einem pH von 9,1, Citrat-Puffer mit einem pH von 7,1, Phosphat-Puffer mit einem pH von 7,4 und Tris/HCl-Puffer mit einem pH von 7,4 gewählt wird, gelingt eine quantitative Extraktion sämtlicher bekannten Mykotoxine aus den agrarischen Produkten, ohne dass für Menschen oder Tiere schädliche Chemikalien eingesetzt werden und gleichzeitig gelingt es mit einer derartigen Verfahrensführung, dass Extraktionen der Mykotoxine aus den Produkten bei Raumtemperatur, d.h. Temperaturen zwischen 15 °C und 25°C durchzuführen, so dass ein rasch durchzuführendes und energiesparendes Verfahren, welches überdies keine für Menschen oder Tiere schädliche Chemikalien benötigt, zur Verfügung gestellt werden kann.

Wenn, wie dies einer Weiterbildung der Erfindung entspricht, das Verfahren so geführt wird, das der Extraktionspuffer in einer Konzentration zwischen 10 mM und 100 mM eingesetzt wird, kann sichergestellt werden, dass die Mykotoxine quantitativ in Lösung gelangen und somit kann ein qualitativer Nachweis auf das Vorhandensein derselben sowie ein quantitativer Nachweis betreffend den Gehalt an Kontaminanten durchgeführt werden, so dass vor Ort entschieden werden kann, ob beispielweise eine Charge von angelieferten agrarischen Produkten einer weiteren Mykotoxine entfernenden Behandlung unterzogen werden muss oder ob sie unmittelbar weiter verwendet werden kann, da beispielsweise die Mykotoxin- oder Kontaminantenbelastung so niedrig ist, dass sie unter einem für Menschen oder Tiere schädlichen Bereich liegt.

Indem, wie dies einer Weiterbildung der Erfindung entspricht, das Verfahren so geführt wird, dass dem Extraktionspuffer ein pulverförmiger Träger beigesetzt wird, insbesondere ein inertes pulverförmiges Silikat, wie hochreine Kieselsäure, gelingt es, die Löslichkeitseigenschaften des Extraktionspuffers gleichbleibend gut zu halten und gleichzeitig die Verarbeitbarkeit des Extraktionspuffers, insbesondere sein gezieltes Einbringen in die in einem Lösungsmittel lösliche Folie, deutlich zu verbessern, wodurch nicht nur die Verarbeitbarkeit verbessert wird, sondern insbesondere die Analystengenauigkeit erhöht werden kann, da auch die Menge an eingesetztem Extraktionspuffer genau quantifizierbar ist.

Indem, wie dies einer Weiterbildung der Erfindung entspricht, dem Extraktionspuffer 0,1 bis 1 % Tensid zugesetzt werden, gelingt eine noch raschere und vollständigere Auflösung der auf den agrarischen Produkten adsorbierten Mykotoxine in dem mit Wasser versetzten Extraktionspuffer. Indem weiters das Tensid aus Polyoxyethylen (23) laurylether (Brij 35), Polyoxyethylen (20) acetylether (Brij 58), 4-(1,1,3,3-tetramethylbutyl)-phenylpolyethylglycol (Triton X100), (1,1,3,3-Tetramethylbutyl)phenyl-polyethylglycol (Triton X114), Polysorbat 20, Polysorbat 80 oder Natriumdodecylsulfat gewählt wird, wird ein besonders rasches in Lösung bringen der Kontaminanten, insbesondere der Mykotoxine sichergestellt, wodurch eine weitere Zeitersparnis erzielt werden kann, um herauszufinden, ob agrarische Produkte oder diese Produkte enthaltende Lebens- bzw. Futtermittel mit Mykotoxinen kontaminiert sind oder nicht.

Eine besonders rasche und zuverlässige Verfahrensführung gelingt dadurch, dass, wie dies einer Weiterbildung der Erfindung entspricht, die In dem Lösungsmittel lösliche Folie als allseitig geschlossener Beutel enthaltend einen Puffer aus wenigstens zwei der folgenden Bestandteile: Natrium oder Kaliumsalze der Borsäure, Carbonsäure, Zitronensäure, Phosphorsäure, phosphoriger Säure, TRIS (Tris(hydroxyxmethyl)-aminomethan) sowie NaCl, wobei der Puffer nach Auflösung eine Molarität von 6 bis 250 mM, insbesondere 10 bis 100 mM, aufweist und der pH-Wert im Bereich von 6,5 - 9,5, insbesondere zwischen 7,1 und 9,1 liegt, eingesetzt wird. Hierbei hat eine pH-Wertabweichung von ± 0,3 keinerlei Auswirkung auf das Testergebnis. Mit einer derartigen Verfahrensführung wird sichergestellt, dass einerseits keine gegebenenfalls schädlichen bzw. nachfolgend schwierig zu entfernenden organischen Lösungsmittel verwendet werden müssen, sondern lediglich auf Wasser basierende Puffersysteme, welche für Menschen und Umgebung unschädlich sind, dass auch die verbrauchten bzw. mit Mykotoxinen beladenen Lösungen einfach aufzubereiten sind, d.h. nicht einer aufwendigen Aufbereitung unterzogen werden müssen, wie dies beispielsweise bei Einsatz von organischen Lösungsmitteln der Fall wäre, dass weiterhin durch Einsatz von allseitig verschlossenen Kunststoffbeuteln, welche in Wasser löslich sind, Chemikalien auch durch ungeübtes Personal sicher und zuverlässig handgehabt und aufgelöst werden können und insbesondere auch die erforderlichen Mengen an Lösungsmitteln, nämlich Wasser beispielsweise einem Aufdruck des Beutels entnommen werden können, wodurch das Verfahren einen extrem geringen Zeitaufwand benötigt. Schließlich ist eine Lagerhaltung von kleinen, dicht verschlossenen Kunststoffbeuteln einfach möglich und bedarf weder eines geschulten Personals noch einer besonderen Vorsicht bei der Lagerhaltung.

Die Erfindung zielt weiterhin darauf ab, eine Vorrichtung zur Verfügung zu stellen, mit welcher es einfach und zuverlässig gelingt, Kontaminanten aus agrarischen bzw. von der Oberfläche agrarischer Produkte zu extrahieren und welche Vorrichtung weder einen übermäßigen Platzbedarf benötigt und überdies auch durch nicht geübtes Personal sicher und zuverlässig bedient werden kann. Zur Lösung dieser Aufgabe ist die erfindungsgemäße Vorrichtung im Wesentlichen dadurch gekennzeichnet, dass der Mischbehälter aus einem zusammenfaltbaren, einen in einer löslichen, insbesondere wasserlöslichen Folie eingeschlossenen Extraktionspuffer umschließenden Kunststoffbeutel gebildet ist. Indem der Mischbehälter aus einem zusammenfaltbaren, einen in einer löslichen, insbesondere wasserlöslichen Folie eingeschlossene Extraktions puffer umschließenden Kunststoffbeutel gebildet ist, wird eine Vorrichtung zur Verfügung gestellt, in welcher in einem Beutel Extraktionspuffer enthalten ist und welche Vorrichtung auch zusammenfaltbar ausgebildet ist. Bei einer derartigen Vorrichtung kann unmittelbar in den Kunststoffbehälter bzw. Kunststoffbeutel das Lösungsmittel eingefügt werden und zusätzlich die zu untersuchenden agrarischen Produkte hinzugefügt werden, so dass beispielsweise die Vorratshaltung von Gefäßen, wie Bechern, Gläsern, Erlenmeyer-Kolben und dgl., in welchen üblicherweise derartige Untersuchungen ausgeführt werden, obsolet wird.

Indem, wie dies einer Weiterbildung der Erfindung entspricht, der Kunststoffbeutel an einem Ende mit ein Aufreißelement aufweisenden Schweißnähten verschlossen ist und weiterhin der in der wasserlöslichen Folie eingeschlossene Extraktionspuffer in dem verschlossenen Kunststoffbeutel aufgenommen ist, wird überdies sichergestellt, dass der In der wasserlöslichen Folie eingeschlossene Extraktionspuffer nicht vorzeitig mit Feuchtigkeit in Kontakt gelangt und beispielsweise vorzeitig aufgelöst wird. Indem weiterhin der Kunststoffbeutel an einem Ende mit einem Aufreißelement versehen ist, kann durch einfaches Aufreißen des Kunststoffbeutels der erforderliche Mischbehälter zur Verfügung gestellt werden, welcher bereits eine für die Größe des Behälters, insbesondere Beutels geeignete Menge an Extraktionspuffer in der wasserlöslichen Folie eingeschlossen aufweist. Indem, wie dies einer Weiterbildung der Erfindung entspricht, die Vorrichtung so ausgebildet ist, dass ein Ende des Kunststoffbeutels mit einem doppelten Boden ausgebildet ist, und dass die zum Inneren des Kunststoffbeutels gerichtete, einen doppelten Boden des Kunststoffbeutels bildende Folie die den Extraktionspuffer einschließende wasserlösliche Folie ist, wird eine besonders einfach handzuhabende Vorrichtung zur Verfügung gestellt, in welche nur mehr Wasser und die zu untersuchenden agrarischen Produkte eingefüllt werden müssen, um eine Untersuchung auf das Vorhandensein von Kontaminanten, insbesondere Mykotoxinen auf der Oberfläche der agrarischen Produkte durchführen zu können.

Gemäß einer Weiterbildung der Erfindung ist die Vorrichtung so ausgebildet, dass das Aufreißelement als Kunststoffstäbchen, insbesondere Rührstäbchen ausgebildet ist, wodurch durch einfaches Rühren im Inneren des Kunststoffbeutels nach Hinzufügen von Wasser bzw. nach Hinzufügen von agrarischen Produkten eine vollständige und sichere Auflösung des Extraktionspuffers und eine Ablösung der gegebenenfalls auf den agrarischen Produkten vorhandenen Mykotoxine gewährleistet wird.

Zur Vereinfachung der Arbeitsweise und insbesondere, um auch ungeübtem Personal auch eine korrekte Untersuchung zu ermöglichen, ist gemäß einer Weiterbildung der Erfindung der Kunststoffbeutel in an sich bekannter Weise mit einer Skalierung versehen. Durch das Vorsehen einer derartigen Skalierung gelingt es auch ungeübtem Personal, exakt die richtige Menge an Lösungsmittel in den Kunststoffbehälter einzufügen, um eine zuverlässige und insbesondere auch quantitative Untersuchung durchzuführen.

Die Erfindung wird nachfolgend anhand von in der Zeichnung dargestellten Ausführungsbeispielen näher erläutert. In dieser zeigt:
Fig. 1 eine erfindungsgemäße Vorrichtung enthaltend einen in einer wasserlöslichen Folie eingeschlossenen Extraktionspuffer,
Fig. 2 eine andere Ausbildung der Vorrichtung von Fig. 1, in welcher der Extraktionspuffer im doppelten Boden eines Kunststoffbeutels vorgesehen ist, und
Fig. 3 die Vorrichtung von Fig. 1 in geöffnetem Zustand im Einsatz.

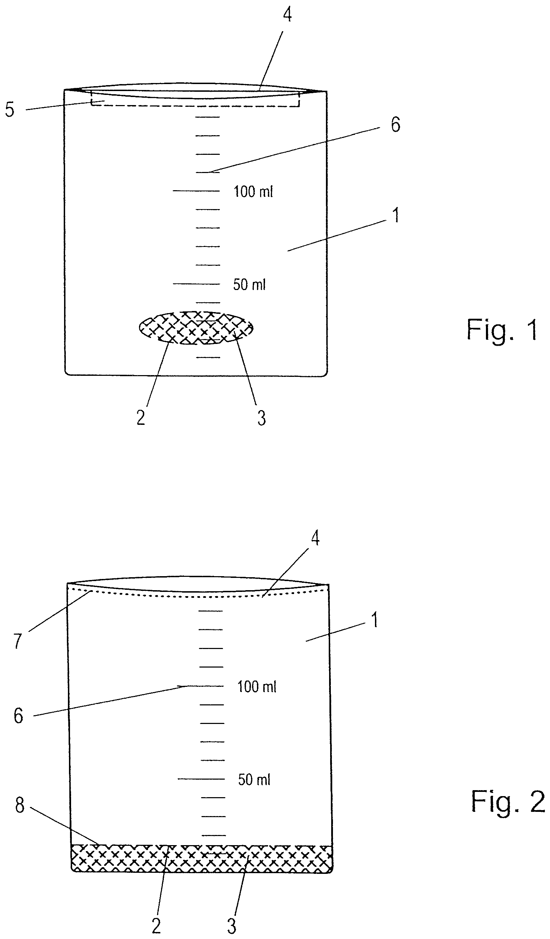

Im Einzelnen ist in Fig. 1 ein Mischbehälter in Form eines Kunststoffbeutels 1 dargestellt, in dessen Inneren ein in einer wasserlöslichen Folie 2 aufgenommener Extraktionspuffer 3 enthalten ist. Der Extraktionspuffer kann hierbei mit einer hochreinen Kieselsäure als Träger versetzt sein, wodurch seine Abfüllung und Homogenisierung verbessert wird, seine Extraktionseigenschaften aber nicht beeinflusst werden. Der Beutel 1 ist in der Darstellung von Fig. 1 im geschlossenen Zustand gezeigt und an seinem oberen Ende ist ein Aufreißelement 4 vorgesehen, in welchem ein stäbchenförmiges Element 5 eingeschlossen ist, welches stäbchenförmige Element 5 beim Öffnen des Beutels 1 einfach mit abgerissen wird und anschließend aus dem abgerissenen Teil des Aufreißelements 4 entnommen werden kann und z.B. als Rührstäbchen verwendet werden kann. Weiterhin weist der Beutel 1 an seiner Außenseite eine Skalierung 6 auf, mit welcher im Einsatz die exakte Menge an einzufüllendem Wasser bestimmt werden kann.

In Fig. 2 ist eine andere Variante einer Vorrichtung gemäß der Erfindung dargestellt, in welcher so weit als möglich die Bezugszeichen beibehalten sind. Der Beutel 1 ist wiederum mit einer Skalierung 6 versehen und weist überdies an seinem oberen Ende ein als Aufreißkante 4 ausgebildetes Aufreißelement 4 auf. Das Aufreißelement 4 in der Darstellung gemäß Fig. 2 ist mit einer Perforierung 7 versehen, wobei beim Öffnen des Beutels 1 einfach der Beutel 1 durch Abreißen des oberen Aufreißelements 4 entlang der Perforierung 7 geöffnet werden kann. Weiterhin ist der untere Bereich des Beutels 1 mit einem doppelten Boden 8 ausgebildet, wobei zwischen dem unteren Ende des Beutels 1 und dem doppelten Boden 8 der Extraktionspuffer 3 eingeschlossen ist. Im Einsatz wird ein derartiger Beutel 1 geöffnet, Wasser bis zur gewünschten Markierung eingefüllt und entweder durch vorsichtiges Schütteln des Beutels oder durch Rühren im Inneren desselben die lösbare Folie 2, welche den Extraktionspuffer 3 als doppelter Boden 8 abdeckt, auflöst, so dass der Puffer mit dem Lösungsmittel in Kontakt gelangt. Nach einem Auflösen des Extraktionspuffers 3 wird in das Innere des Beutels 1 das zu untersuchende agrarische Produkt 9 eingefüllt und entweder ein paar Minuten stehen gelassen oder weitergerührt, um möglichst rasch die auf der Oberfläche des Produkts 9 enthaltenen Kontaminanten zu lösen. Selbstverständlich können auch das agrarische Produkt und der aufzulösende Puffer zeitgleich oder in beliebiger Reihenfolge eingefüllt werden. In weiterer Folge genügt es, beispielsweise mit einem Streifentest nachzuweisen, ob, bzw. im Falle eines quantitativen Streifentests, welche Menge und/oder Arten Kontaminanten bzw. Mykotoxine auf der Oberfläche des agrarischen Produkts 9 vorgelegen sind.

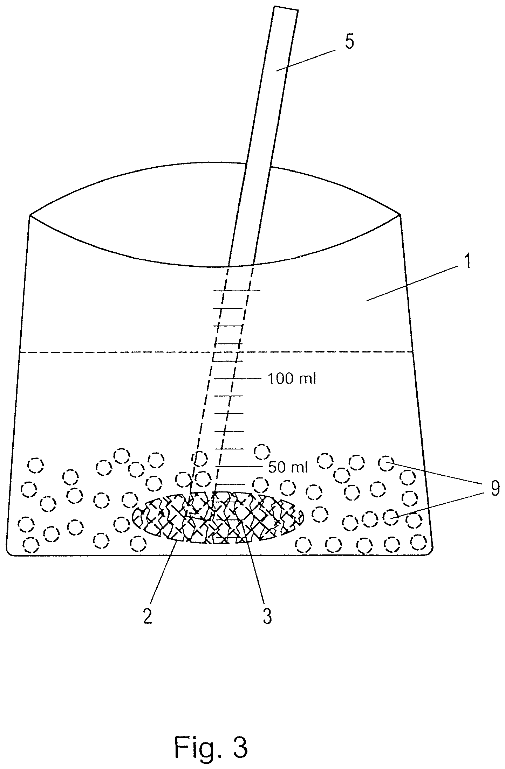

In Fig. 3 ist der Beutel 1 von Fig. 1 gezeigt, wobei er in dieser Darstellung im aufgerissenen Zustand gezeigt ist und sowohl der Extraktionspuffer 3 sich bereits in Auflösung befindet, da die wasserlösliche Folie 2 bereits Risse und Zerstörungen aufweist und überdies die zu untersuchenden agrarischen Produkte 9 ebenso wie das Lösungsmittel Wasser eingefüllt wurden. Zum Auflösen bzw. Erleichtern des Auflösens des Extraktionspuffers 3 bzw. um die Kontaminanten von der Oberfläche der agrarischen Produkte 9 abzulösen, wird hierbei mit dem Rührstäbchen 5 im Inneren des Beutels 1 gerührt.

Nach vollständiger Auflösung des Extraktionspuffers 3 kann z.B. mit einem herkömmlichen Streifentest auf das Vorhandensein von Kontaminanten untersucht werden.

### Beispiel 1: Wiederfindung verschiedener Mykotoxine in Maisproben:

Die nachfolgenden Tabellen zeigen die Wiederfindung der verschiedenen Mykotoxine in Maisproben, wenn sie mit verschiedenen Puffern bestehend aus Salzen und Tensiden getestet werden, sowie eine Zusammenfassung der Wiederfindung der Toxine im Phosphat-puffer-System (jeweils Konzentration 10 mM und 100 mM), durchgeführt mit ELISA Tests:
- Carbonat Puffer, pH 8,5,
- Carbonat Puffer, pH 9,1,
- Citrat Puffer, pH 7,1,
- Phosphat Puffer, pH 7,4 und
- Tris/HCl Puffer, pH 7,5.

In den Tabellen sind folgende Schlüssel für die Wiederfindung angegeben:

| **Ergebnis** | **Wiederfindung** |
|---|---|
| (+) | < 30 % |
| + | 30-50 % |
| ++ | 50-80 % |
| +++ | > 80 % |

| | |
|---|---|
| Ergebnisse der Streifentests (LFDs): | |

### Aflatoxine:

Tabelle 1 zeigt die Wiederfindungen von Aflatoxinen in Maisproben. Verschiedene Pufferkombinationen (Salz+Tensid) wurden getestet.

**Tabelle 1: Aflatoxine**

| **Puffer [mM]** | **Tween 20 [%] 0,1/1** | **Tween 80 [%] 0,1/1** | **Triton X100 [%] 0,1/1** | **Triton X114 [%] 0,1/1** | **Brij 35 [%] 0,1/1** | **Brij 58 [%] 0,1/1** | **SDS [%] 0,1/1** |
|---|---|---|---|---|---|---|---|
| Borat 10 mM | ++/++ | ++/+++ | ++/++ | ++/+++ | ++/++ | ++/+++ | +++/++ |
| Borat 100 mM | ++/++ | ++/++ | ++/++ | ++/++ | ++/++ | ++/++ | +++/++ |
| Carbonat 10 mM | ++/+++ | ++/+++ | ++/++ | ++/+++ | ++/++ | ++/+++ | +++/++ |
| Carbonat 100 mM | ++/+++ | ++/+++ | ++/++ | ++/+++ | ++/++ | ++/++ | +++/++ |
| Citrat 10 mM | +/+ | +/++ | +/++ | +/++ | +/++ | +/++ | ++/+ |
| Citrat 100 mM | +/+ | +/+ | +/+ | +/+ | +/+ | +/+ | ++/+ |
| Phosphat 10 mM | ++/+++ | ++/+++ | ++/++ | ++/++ | ++/+++ | ++/+++ | +++/++ |
| Phosphat 100 mM | ++/+++ | ++/++ | ++/++ | ++/++ | ++/+++ | ++/+++ | +++/++ |
| Tris/HCl 10 mM | ++/++ | ++/++ | ++/++ | ++/++ | ++/++ | ++/++ | +++/++ |
| Tris/HCl 100 mM | ++/++ | ++/++ | ++/++ | ++/++ | ++/++ | ++/++ | +++/++ |

### Deoxynivalenol:

Tabelle 2 zeigt die Wiederfindungen von Deoxynivalenol in Maisproben. Verschiedene Pufferkombinationen (Salz+Tensid) wurden getestet.

**Tabelle 2: Deoxynivalenol**

| **Puffer [mM]** | **Tween 20 [%] 0,1/1** | **Tween 80 [%] 0,1/1** | **Triton X100 [%] 0,1/1** | **Triton X114 [%] 0,1/1** | **Brij 35 [%] 0,1/1** | **Brij 58 [%] 0,1/1** | **SDS [%] 0,1/1** |
|---|---|---|---|---|---|---|---|
| Borat 10 mM | +++/+++ | +++/+++ | +++/+++ | +++/+++ | +++/+++ | +++/+++ | +++/++ |
| Borat 100 mM | +++/+++ | +++/+++ | +++/+++ | +++/+++ | +++/+++ | +++/+++ | +++/++ |
| Carbonat 10 mM | +++/+++ | +++/+++ | +++/+++ | +++/+++ | +++/+++ | +++/+++ | +++/++ |
| Carbonat 100 mM | +++/+++ | +++/+++ | +++/+++ | +++/+++ | +++/+++ | +++/+++ | +++/++ |
| Citrat 10 mM | ++/++ | ++/++ | ++/++ | ++/++ | ++/++ | ++/++ | ++/+ |
| Citrat 100 mM | ++/++ | ++/++ | ++/++ | ++/++ | ++/++ | ++/++ | ++/+ |
| Phosphat 10 mM | +++/+++ | +++/+++ | +++/+++ | +++/+++ | +++/+++ | +++/+++ | +++/++ |
| Phosphat 100 mM | +++/+++ | +++/+++ | +++/+++ | +++/+++ | +++/+++ | +++/+++ | +++/++ |
| Tris/HCl 10 mM | +++/+++ | +++/+++ | +++/+++ | +++/+++ | +++/+++ | +++/+++ | +++/++ |
| Tris/HCl 100 mM | +++/+++ | +++/+++ | +++/+++ | +++/+++ | +++/+++ | +++/+++ | +++/++ |

### Fumonisine:

Tabelle 3 zeigt die Wiederfindungen von Fumonisinen in Maisproben. Verschiedene Pufferkombinationen (Salz+Tensid) wurden getestet.

**Tabelle 3: Fumonisine**

| **Puffer [mM]** | **Tween 20 [%] 0,1/1** | **Tween 80 [%] 0,1/1** | **Triton X100 [%] 0,1/1** | **Triton X114 [%] 0,1/1** | **Brij 35 [%] 0,1/1** | **Brij 58 [%] 0,1/1** | **SDS [%] 0,1/1** |
|---|---|---|---|---|---|---|---|
| Borat 10 mM | ++/+++ | ++/+++ | ++/+++ | ++/+++ | ++/(+) | ++/(+) | ++/(+) |
| Borat 100 mM | ++/+++ | ++/+++ | ++/+++ | ++/+++ | ++/(+) | ++/(+) | ++/(+) |
| Carbonat 10 mM | ++/+++ | ++/+++ | ++/+++ | ++/+++ | ++/(+) | ++/(+) | ++/(+) |
| Carbonat 100 mM | ++/+++ | ++/+++ | ++/+++ | ++/+++ | ++/(+) | ++/(+) | ++/(+) |
| Citrat 10 mM | ++/++ | ++/++ | ++/++ | ++/++ | ++/(+) | ++/(+) | ++/(+) |
| Citrat 100 mM | ++/++ | ++/++ | ++/++ | ++/++ | ++/(+) | ++/(+) | ++/(+) |
| Phosphat 10 mM | ++/+++ | ++/+++ | ++/+++ | ++/+++ | ++/(+) | ++/(+) | ++/(+) |
| Phosphat 100 mM | ++/+++ | ++/+++ | ++/+++ | ++/+++ | ++/(+) | ++/(+) | ++/(+) |
| Tris/HCl 10 mM | ++/+++ | ++/+++ | ++/+++ | ++/+++ | ++/(+) | ++/(+) | ++/(+) |
| Tris/HCl 100 mM | ++/+++ | ++/+++ | ++/+++ | ++/+++ | ++/(+) | ++/(+) | ++/(+) |

### Zearalenone:

Tabelle 4 zeigt die Wiederfindungen von Zearalenon in Maisproben. Verschiedene Pufferkombinationen (Salz+Tensid) wurden getestet.

**Tabelle 4: Zearalenon:**

| **Puffer [mM]** | **Tween 20 [%] 0,1/1** | **Tween 80 [%] 0,1/1** | **Triton X100 [%] 0,1/1** | **Triton X114 [%] 0,1/1** | **Brij 35 [%] 0,1/1** | **Brij 58 [%] 0,1/1** | **SDS [%] 0,1/1** |
|---|---|---|---|---|---|---|---|
| Borat 10 mM | +++/++ | +++/++ | +++/++ | +++/++ | +++/++ | +++/++ | +++/+ |
| Borat 100 mM | +++/++ | +++/++ | +++/++ | +++/++ | +++/++ | +++/++ | +++/+ |
| Carbonat 10 mM | ++/+++ | ++/+++ | +++/++ | ++/+++ | +++/++ | ++/+++ | +++/+ |
| Carbonat 100 mM | ++/+++ | ++/+++ | +++/++ | ++/+++ | +++/++ | ++/+++ | +++/+ |
| Citrat 10 mM | +/+ | +/+ | +/+ | +/+ | +/+ | +/+ | ++/+ |
| Citrat 100 mM | +/+ | +/+ | +/+ | +/+ | +/+ | +/+ | ++/+ |
| Phosphat 10 mM | +++/++ | +++/++ | +++/++ | +++/++ | +++/++ | +++/++ | +++/+ |
| Phosphat 100 mM | +++/++ | +++/++ | +++/++ | +++/++ | +++/++ | +++/++ | +++/+ |
| Tris/HCl 10 mM | ++/+++ | ++/+++ | ++/+++ | ++/+++ | +++/++ | ++/+++ | +++/+ |
| Tris/HCl 100 mM | ++/+++ | ++/+++ | ++/+++ | ++/+++ | +++/++ | ++/+++ | +++/+ |

**Tabelle 5: Übersicht LFD Ergebnisse:**

| **TOXIN PBS Puffer** | **Tween 20 [%] 0,1/1** | **Tween 80 [%] 0,1/1** | **Triton X100 [%] 0,1/1** | **Triton X114 [%] 0,1/1** | **Brij 35 [%] 0,1/1** | **Brij 58 [%] 0,1/1** | **SDS [%] 0,1/1** |
|---|---|---|---|---|---|---|---|
| Alfatoxine 10 mM | ++/++ | ++/++ | ++/++ | ++/++ | ++/+++ | ++/++ | +++/+ |
| Alfatoxine 100 mM | ++/++ | ++/++ | ++/++ | ++/++ | ++/+++ | ++/++ | +++/+ |
| Deoxynivalenol 10 mM | +++/+ | +++/+ | +++/++ | +++/+ | +++/++ | +++/+ | +++/+ |
| Deoxynivalenol 100 mM | +++/+ | +++/+ | +++/++ | +++/+ | +++/++ | +++/+ | +++/+ |
| Fumonisine 10 mM | ++/++ | ++/++ | ++/+++ | ++/++ | ++/(+) | ++/(+) | ++/(+) |
| Fumonisine 100 mM | ++/++ | ++/++ | ++/+++ | ++/++ | ++/(+) | ++/(+) | ++/(+) |
| Zearalenone 10 mM | ++/+++ | +++/+ | +++/++ | +++/+ | +++/++ | ++/+ | +++/+ |
| Zearalenone 100 mM | ++/+++ | +++/+ | +++/++ | +++/+ | +++/++ | ++/+ | +++/+ |

Aus diesen Tabellen folgt, dass die LFD und ELISA Ergebnisse vergleichbar sind und die verschiedenen Puffersysteme wie auch verschiedene Tenside und Konzentrationen für die Extraktionsmethode anwendbar sind und zuverlässige Ergebnisse liefern.

### Beispiel 2: Extraktion von Lebensmittelallergenen mit in wasserlöslichen Folien enthaltenem Extraktionspuffer:

Tris/HCl und ein PBS-Puffer, die jeweils in einer wasserlöslichen Folie eingeschweißt sind, werden in einen Kunststoffbehälter eingebracht und mit kaltem Leitungswasser versetzt, bis jeweils 100 mM, 150 mM, 200 mM und 250 mM Lösungen gebildet wurden. Die Lösungen hatten in Abhängigkeit von dem eingesetzten Puffer pH-Werte zwischen 6,5 und 8,8.

Mit beiden Extraktionspuffern gelang es, mit allen vier verschiedenen Konzentrationen die folgenden Lebensmittelallergene quantitativ zu extrahieren: β-Lactoglobulin, Kasein, Cashewnuss, Eiweiss, Erdnuss, Fisch, Haselnuss, Krustentiere, Lupin, Mandel, Milch, Ovalbulmin, Pistazie, Senf, Sesam, Soja, Walnuss.

### Beispiel 3: Extraktion von genetisch veränderten Organismen mit in wasserlöslichen Folien enthaltenem Extraktionspuffer:

Tris/HCl, PBS und Borat-Puffer, die jeweils gemeinsam mit Tween oder Triton und Silikagel in einer wasserlöslichen Folie eingeschweißt sind, werden in einen Kunststoffbehälter eingebracht und mit kaltem Leitungswasser versetzt, bis jeweils 100 mM, 150 mM, 200 mM und 250 mM Lösungen gebildet wurden.

Mit beiden Extraktionspuffern gelang es, mit allen vier verschiedenen Konzentrationen die folgenden genetisch veränderten Organismen quantitativ zu extrahieren und die Ergebnisse mittels Statfax® und Chromate®-Lesegeräten zu quantifizieren: RUR, Cry1F, Cry1AB, Cry3bb, LL, Cry34AB1, VIP, Btk, BR, B2R, EPSPS, DAS Cry1Ac.

## Patentansprüche

1. Verfahren zur Extraktion von Kontaminanten aus agrarischen Produkten (9) oder agrarische Produkte enthaltenden Lebens- oder Futtermitteln, umfassend ein in Kontakt bringen eines Lösungsmittels für die Kontaminanten mit den die Kontaminanten angelagert aufweisenden bzw. enthaltenden agrarischen Produkten (9) oder den Lebens- oder Futtermitteln, sowie wenigstens einen Extraktionspuffer (3) und gegebenenfalls einen pulverförmigen Träger in einem gegebenenfalls verschießbaren Mischbehälter, **dadurch gekennzeichnet, dass** der in einer in dem Lösungsmittel löslichen Folie (2) enthaltene, mit wenigstens einem insbesondere nicht ionischen Tensid versetzte Extraktionspuffer (1) sowie das kontaminierte agrarische Produkt (9) oder Lebens- oder Futtermittel mit kaltem Wasser als Lösungsmittel in dem Mischbehälter in Kontakt gebracht werden, und der Extraktionspuffer (3) sowie die Kontaminanten durch Schütteln oder Rühren in dem Mischbehälter in Lösung gebracht werden.

2. Verfahren nach Anspruch 1, **dadurch gekennzeichnet, dass** Wasser mit einer Temperatur zwischen 8 und 25°C, insbesondere destilliertes Wasser eingesetzt wird.

3. Verfahren nach Anspruch 1 oder 2, **dadurch gekennzeichnet, dass** als Extraktionspuffer (3) wenigstens ein Puffer gewählt aus der Gruppe der Borat-Puffer mit einem pH von 8,5, Carbonat-Puffer mit einem pH von 9,1, Citrat-Puffer mit einem pH von 7,1, Phosphat-Puffer mit einem pH von 7,4 und Tris/HCl-Puffer mit einem pH von 7,4 gewählt wird.

4. Verfahren nach Anspruch 1, 2 oder 3, **dadurch gekennzeichnet, dass** der Extraktionspuffer (3) in einer Konzentration zwischen 10 mM und 100 mM eingesetzt wird.

5. Verfahren nach einem der Ansprüche 1 bis 4, **dadurch gekennzeichnet, dass** dem Extraktionspuffer (3) 0,1 bis 1 % Tensid zugesetzt werden.

6. Verfahren nach einem der Ansprüche 1 bis 5, **dadurch gekennzeichnet, dass** als pulverförmiger Träger ein inertes pulverförmiges Silikat, wie hochreine Kieselsäure eingesetzt wird,

7. Verfahren nach Anspruch 6, **dadurch gekennzeichnet, dass** das Tensid gewählt aus Polyoxyethylen (23) laurylether (Brij 35), Polyoxyethylen (20) acetylether (Brij 58), 4-(1,1,3,3-tetramethylbutyl)-phenyl-polyethylglycol (Triton X100), (1,1,3,3-Tetramethylbutyl)phenyl-polyethylglycol (Triton X114), Polysorbat 20, Polysorbat 80 oder Natriumdodecylsulfat eingesetzt wird.

8. Verfahren nach einem der Ansprüche 1 bis 7, **dadurch gekennzeichnet, dass** die in dem Lösungsmittel lösliche Folie (2) als allseitig geschlossener Beutel (1) enthaltend einen Puffer aus wenigstens zwei der folgenden Bestandteile: Natrium oder Kaliumsalze der Borsäure, Carbonsäure, Zitronensäure, Phosphorsäure, phosphoriger Säure, TRIS (Tris(hydroxyxmethyl)-aminomethan) sowie NaCl, wobei der Puffer nach Auflösung eine Molarität von 6 bis 250 mM, insbesondere 10 bis 100 mM, aufweist und der pH-Wert im Bereich von 6,5 - 9,5, insbesondere zwischen 7,1 und 9,1 liegt, eingesetzt wird.

9. Vorrichtung zum Extrahieren von Kontaminanten aus agrarischen bzw. von der Oberfläche agrarischer Produkte (9) oder agrarische Produkte enthaltenden Lebens- oder Futtermitteln, umfassend einen gegebenenfalls verschließbaren Mischbehälter, **dadurch gekennzeichnet, dass** der Mischbehälter aus einem zusammenfaltbaren, einen in einer löslichen, insbesondere wasserlöslichen Folie (2) eingeschlossenen Extraktionspuffer (3) umschließenden Kunststoffbeutel (1) gebildet ist.

10. Vorrichtung nach Anspruch 9, **dadurch gekennzeichnet, dass** der Kunststoffbeutel (1) mit insbesondere an einem Ende mit ein Aufreißelement (4) aufweisenden Schweißnähten verschlossen ist und dass der in der wasserlöslichen Folie (2) eingeschlossene Extraktionspuffer (3) in dem verschlossenen Kunststoffbeutel (1) aufgenommen ist.

11. Vorrichtung nach Anspruch 9 oder 10, **dadurch gekennzeichnet, dass** ein Ende des Kunststoffbeutels (1) mit einem doppelten Boden (8) ausgebildet ist, und dass die zum Inneren des Kunststoffbeutels (1) gerichtete, einen doppelten Boden (8) des Kunststoffbeutels (1) bildende Folie (2) die den Extraktionspuffer (3) einschließende wasserlösliche Folie (2) ist.

12. Vorrichtung nach einem der Ansprüche 9 bis 11, **dadurch gekennzeichnet, dass** das Aufreißelement (4) als Kunststoffstäbchen, insbesondere Rührstäbchen ausgebildet ist.

13. Vorrichtung nach einem der Ansprüche 9 bis 12, **dadurch gekennzeichnet, dass** in an sich bekannter Weise der Kunststoffbeutel (1) eine Skalierung (6) aufweist.

## Claims

1. A method for extracting contaminants from agrarian products (9) or foods or animal feeds containing agrarian products, comprising contacting a solvent for the contaminants with the agrarian products (9) containing or having adsorbed the contaminants, or the foods or animal feeds, and at least one extraction buffer (3), and optionally a powdery carrier, in an optionally closable mixing vessel, **characterized in that** the extraction buffer (3), which is contained in a film (2) soluble in the solvent and is supplemented with at least one surfactant, in particular nonionic surfactant, and the contaminated agrarian product (9) or food or animal feed are contacted with cold water as solvent in the mixing vessel, and the extraction buffer (3) and the contaminants are brought into solution by agitating or stirring in the mixing vessel.

2. A method according to claim 1, **characterized in that** water having a temperature between 8°C and 25°C, in particular distilled water, is used.

3. A method according to claim 1 or 2, **characterized in that** at least one buffer selected from the group consisting of borate buffers having a pH of 8.5, carbonate buffers having a pH of 9.1, citrate buffers having a pH of 7.1, phosphate buffers having a pH of 7.4 and Tris/HCl buffer having a pH of 7.4 is used as extraction buffer (3).

4. A method according to claim 1, 2 or 3, **characterized in that** the extraction buffer (3) is used at a concentration of between 10 mM and 100 mM.

5. A method according to any one of claims 1 to 4, **characterized in that** 0.1 to 1% of a surfactant is added to the extraction buffer (3).

6. A method according to any one of claims 1 to 5, **characterized in that** an inert silicate powder such as high-purity silicic acid is used as said powdery carrier.

7. A method according to claim 6, **characterized in that** the surfactant is selected from polyoxyethyelene (23) lauryl ether (Brij 35), polyoxyethylene (20) acetyl ether (Brij 58), 4-(1,1,3,3-tetramethylbutyl)phenyl-polyethylene glycol (Triton X100), (1,1,3,3-tetramethylbutyl)phenyl-polyethylene glycol (Triton X114), polysorbate 20, polysorbate 80 or sodium dodecyl sulfate.

8. A method according to any one of claims 1 to 7, **characterized in that** the film (2) soluble in the solvent is used as a bag (1) closed on all sides and containing a buffer comprising at least two of the following components: sodium or potassium salts of boric acid, carboxylic acid, citric acid, phosphoric acid, phosphorous acid, TRIS (Tris(hydroxymethyl)-aminomethane) and NaCl, said buffer after dissolution having a molarity of 6 to 250 mM, in particular 10 to 100 mM, and a pH ranging from 6.5 to 9.5, in particular between 7.1 and 9.1.

9. An apparatus for extracting contaminants from agrarian products (9) or from the surfaces of agrarian products (9), or foods or animal feeds containing agrarian products, including an optionally closable mixing vessel, **characterized in that** the mixing vessel is comprised of a foldable plastic bag (1) containing an extraction buffer (3) enclosed in a soluble, in particular water-soluble, film (2).

10. A device according to claim 9, **characterized in that** the plastic bag (1) is closed by welds comprising, in particular on one end, a tear-open element (4), and that the extraction buffer (3) enclosed in the water-soluble film (2) is received in the closed plastic bag (1).

11. A device according to claim 9 or 10, **characterized in that** one end of the plastic bag (1) is formed with a double bottom (8), and that the film (2) facing the interior of the plastic bag (1) and forming said double bottom (8) of the plastic bag (1) is comprised of the water-soluble film (2) enclosing the extraction buffer (3).

12. A device according to any one of claims 9 to 11, **characterized in that** the tear-open element (4) is designed as a plastic rod, in particular stirring rod.

13. A device according to any one of claims 9 to 12, **characterized in that** the plastic bag (1) comprises a scale (6) in a manner known per se.

## Revendications

1. Procédé servant à l'extraction de contaminants de produits agricoles (9) ou de produits alimentaires et d'aliments pour animaux contenant des produits agricoles, comprenant une mise en contact d'un solvant pour les contaminants avec les produits agricoles (9) présentant ou contenant de manière accumulée les contaminants ou avec les produits alimentaires ou les aliments pour animaux, ainsi qu'au moins un tampon d'extraction (3) et éventuellement un support pulvérulent dans un contenant de mélange pouvant éventuellement être fermé, **caractérisé en ce que** le tampon d'extraction (1) contenu dans un film (2) soluble dans le solvant, mélangé à au moins un tensio-actif en particulier non ionique ainsi que le produit agricole (9) ou le produit alimentaire ou l'aliment pour animaux contaminé sont amenés en contact dans le contenant de mélange avec de l'eau froide en tant que solvant, et le tampon d'extraction (3) ainsi que les contaminants sont dissous dans le contenant de mélange en les secouant ou en les agitant.

2. Procédé selon la revendication 1, **caractérisé en ce que** de l'eau avec une température comprise entre 8 et 25 °C, en particulier de l'eau distillée, est utilisée.

3. Procédé selon la revendication 1 ou 2, **caractérisé en ce qu'**est choisi en tant que tampon d'extraction (3) au moins un tampon choisi parmi le groupe des tampons de borate avec un pH de 8,5, des tampons de carbonate avec un pH de 9,1, des tampons de citrate avec un pH de 7,1, des tampons de phosphate avec un pH de 7,4 et un tampon de Tris/HCI avec un pH de 7,4.

4. Procédé selon la revendication 1, 2 ou 3, **caractérisé en ce que** le tampon d'extraction (3) est utilisé en une concentration comprise entre 10 mM et 100 mM.

5. Procédé selon l'une des revendications 1 à 4, **caractérisé en ce que** 0,1 à 1 % de tensio-actifs sont ajoutés au tampon d'extraction (3).

6. Procédé selon l'une des revendications 1 à 5, **caractérisé en ce qu'**un silicate inerte pulvérulent, tel qu'un acide silicique de pureté élevée, est utilisé en tant que support pulvérulent.

7. Procédé selon la revendication 6, **caractérisé en ce que** le tensio-actif est choisi parmi le polyoxyéthylène (23) lauryléther (Brij 35), le polyoxyéthylène (20) acétyléther (Brij 58), le 4-(1,1,3,3-tétraméthylbutyl)-phényl-polyéthylglycol (Triton X100), le (1,1,3,3-tétraméthylbutyl)phénylpolyéthylglycol (Triton X114), le polysorbate 20, le polysorbate 80 ou le dodécylsulfate de sodium.

8. Procédé selon l'une des revendications 1 à 7, **caractérisé en ce que** le film (2) soluble dans le solvant est utilisé en tant que sachet (1) fermé de tous les côtés contenant un tampon composé d'au moins deux des constituants suivants : le sodium ou des sels de potassium de l'acide borique, l'acide carboxylique, l'acide citrique, l'acide phosphorique, un acide contenant du phosphore, du TRIS (tris(hydroxyméthyl)-aminométhane) ainsi que du NaCl, dans lequel le tampon présente après dissolution une molarité allant de 6 à 250 mM, en particulier de 10 à 100 mM, et la valeur pH se situe dans la plage de 6,5 - 9,5, en particulier est compris entre 7,1 et 9,1.

9. Dispositif servant à extraire des contaminants de produits agricoles ou de la surface de produits agricoles (9) ou de produits alimentaires ou d'aliments pour animaux contenant des produits agricoles, comprenant un contenant de mélange pouvant éventuellement être fermé, **caractérisé en ce que** le contenant de mélange est formé à partir d'un sachet en plastique (1) pouvant être replié, entourant un tampon d'extraction (3) enfermé dans un film (2) soluble, en particulier hydrosoluble.

10. Dispositif selon la revendication 9, **caractérisé en ce que** le sachet en plastique (1) est fermé avec des cordons de soudure présentant en particulier à une extrémité un élément à déchirer (4), et que le tampon d'extraction (3) enfermé dans le film (2) hydrosoluble est logé dans le sachet en plastique (1) fermé.

11. Dispositif selon la revendication 9 ou 10, **caractérisé en ce qu'**une extrémité du sachet en plastique (1) est réalisée avec un double fond (8), et que le film (2) dirigé vers l'intérieur du sachet en plastique (1), formant un double fond (8) du sachet en plastique (1) est le film (2) hydrosoluble enfermant le tampon d'extraction (3).

12. Dispositif selon l'une des revendications 9 à 11, **caractérisé en ce que** l'élément à déchirer (4) est réalisé sous la forme d'un bâtonnet en plastique, en particulier d'un bâtonnet agitateur.

13. Dispositif selon l'une des revendications 9 à 12, **caractérisé en ce que** le sachet en plastique (1) présente d'une manière connue en soi une graduation (6).
